# NOUVEAU FASCICULE DE BREVET EUROPEEN

(11) **EP 1 369 432 B2**
(45) Date de publication et mention de la décision concernant l'opposition: **07.10.2015**
(45) Mention de la délivrance du brevet: 09.08.2006
(21) Numéro de dépôt: 03291325.3
(22) Date de dépôt: 03.06.2003
(51) Int. Cl.: C08B 30/18, C12P 19/18, A61M 1/28

(54) **Polymères solubles de glucose hautement branchés et leur procédé d'obtention**
Lösliche hochverzweigte Glukose-Polymere und Verfahren zu deren Herstellung
Soluble highly branched glucose polymers and process for their preparation

(30) Priorité: 06.06.2002 FR 0206952
(43) Date de publication de la demande: 10.12.2003
(73) Titulaire: Roquette Frères, 62136 Lestrem (FR)
(72) Inventeur: Backer, Daniel, 62350 Saint-Venant (FR); Saniez, Marie-Hélène, 59350 Saint Andre (FR)
(74) Mandataire: Cabinet Plasseraud

(56) Documents cités:
- EP-A- 0 115 911
- EP-A- 1 006 128
- EP-A1- 0 690 170
- WO-A-82/03329
- WO-A2-01//64933
- FR-A- 2 792 941
- US- - 3 974 032
- US-A- 4 454 161
- US-A- 5 116 969
- US-A- 5 612 202
- US-A- 5 837 060
- US-A- 5 886 168
- US-A- 5 929 052
- US-A1-2002/0 065 410
- PATENT ABSTRACTS OF JAPAN vol. 2002, no. 2, 2 avril 2002 (2002-04-02) & JP 2001 294601 A (AKITA PREFECTURE), 23 octobre 2001 (2001-10-23) & DATABASE WPI Week 200218 Derwent Publications Ltd., London, GB; AN 2002-134411
- A. REINER: 'Analyse der Verzweigungsheterogenität von Glykogen mit Enzymatischem Abbau, Lichtstreuung und Modellrechnung' INAUGURAL-DISSERTATION Juin 1981, pages 1 - 68
- J. HELLER ET AL.: 'Biochimica et Biophysica Acta', vol. 81, 1964 pages 96 - 100
- L.M. MARCHAL ET AL.: 'The Use of Freezing-Point Depression for the Theoretical Dextrose Equivalent Measurement' STARCH/STÄRKE vol. 48, no. 6, 1996, pages 220 - 224
- Reproduction de l'exemple 1 de D2
- Reproduction de l'exemple 3 de D2
- Reproduction de l'exemple 3 de D2
- Autorisation pour le Voluven HES 130/0,4
- Résultat de l'analyse HES 130/0,4
- Facture du 27.03.02 Voluven
- Certificat d'analyse HES 130/0,4
- W. FOERST, DR. DR. H.C.: 'Ullmanns Encyklopädie der technischen Chemie', vol. 3, 1965, URBAN & SCHWARZENBERG, MÜNCHEN - BERLIN page 34
- R. L. WHISTLER ET AL.: 'Starch Chemistry and Technology', vol. 2, 1984, ACADEMIC PRESS, INC., ORLANDO - SAN DIEGO pages 346 - 347
- Exemples 1 et 3 de D2 versus Reproduction des exemples 1 et 3 de D2 par l'Opposant
- Roquette Frères Jet Cooker, 2008
- Manuel d'utilisation de l'osmomètre Fiske Mark 3, 1997

## Description

L'invention a pour objet des polymères solubles de glucose hautement branchés ayant une teneur en sucres réducteurs inférieure à 1 % et présentant un taux de liaisons glucosidiques α-1,6 remarquablement élevé, compris entre 12 et 30 %, pour une distribution de poids moléculaire très étroite, comprise entre 0,3. 10⁵ et 2. 10⁵ daltons et une très faible osmolalité, comprise entre 1 et 15 mOsm/kg.

Ces polymères solubles de glucose branchés présentent par ailleurs une faible viscosité et une absence de rétrogradation, même après stockage à froid après de longues périodes de temps.

L'invention concerne également un procédé de fabrication desdits polymères solubles de glucose hautement branchés.

Elle vise encore des compositions comprenant de tels polymères solubles de glucose branchés qu'il est possible d'utiliser dans de nombreuses applications industrielles et notamment dans les industries alimentaires et surtout pharmaceutiques.

Les polymères de glucose classiquement accessibles industriellement sont notamment préparés par hydrolyse des amidons naturels ou hybrides et de leurs dérivés.

Les hydrolysats d'amidon standard sont ainsi produits par hydrolyse acide ou enzymatique d'amidon de céréales ou de tubercules. Ils sont en fait un mélange de glucose et de polymères du glucose, de poids moléculaires extrêmement variés.

Ces hydrolysats d'amidon (dextrines, maltodextrines...) produits dans l'industrie (avec un certain Degré de Polymérisation ou DP moyen) consistent en une large distribution de saccharides contenant à la fois des structures linéaires (liaisons glucosidiques α-1,4) et branchées (liaisons glucosidiques α-1,6).

Ces hydrolysats d'amidon, et notamment les maltodextrines, sont utilisés comme transporteur ou agent de charge, agent texturant, support d'atomisation, agent de remplacement de matières grasses, agent filmogène, agent de contrôle de congélation, agent anti-cristallisant, ou pour leur apport nutritionnel.

Il est connu par ailleurs de l'homme du métier que la composition saccharidique des maltodextrines détermine à la fois leurs propriétés physiques et biologiques.

C'est ainsi que leur hygroscopicité, leur fermentescibilité dans les produits alimentaires, leur viscosité, leur caractère édulcorant, leur stabilité, leur caractère gélifiant et leur osmolalité sont des critères classiquement déterminés pour leurs différents domaines d'application.

Les connaissances de base du comportement physico-chimique de ces saccharides conduisent ainsi à les intégrer par exemple dans les boissons pour sportifs, les boissons liquides à solubilité limitée, les liquides parentéraux et entéraux ou dans des aliments pour diabétiques.

De ce fait, pour ces différentes applications, diverses propriétés physiques et biologiques sont requises.

Il est par exemple connu que le taux d'absorption de ces saccharides est déterminé par le taux de vidange gastrique et le taux d'adsorption intestinal, dont le contrôle est assuré par l'osmolalité desdits saccharides.

Au niveau intestinal, les maltodextrines sont hydrolysées par l'α-amylase pancréatique, ce qui conduit à réduire leur taille jusqu'aux dextrines limites, et un certain nombre d'enzymes liées à la muqueuse intestinale (maltase, sucrase et α-dextrinase) continuent à hydrolyser les saccharides linéaires et branchés en glucose.

Si le glucose passe facilement la barrière intestinale (diffusion passive), il n'en est pas de même pour les saccharides de faible DP. C'est ainsi que des oligosaccharides linéaires seront adsorbés plus vite que des oligosaccharides branchés, bien que le maltose et le maltotriose soient absorbés plus vite que le glucose.

Les bactéries du colon vont fermenter tous les hydrates de carbone qui ne sont pas adsorbés par l'intestin grêle. La fermentation excessive par ces bactéries amènera des désordres intestinaux tels les crampes et la flatulence.

Il est également connu que l'osmolalité influence le taux d'absorption / sécrétion d'eau dans l'intestin grêle. Plus l'osmolalité d'un composé est élevée, plus elle induit une entrée de fluide dans l'intestin et conduit à des dérèglements graves de l'intestin (diarrhée osmotique), avec perte concomitante de fluides et d'électrolytes.

L'osmolalité d'une solution est égale à la quantité de moles dissoutes par kg d'eau, impliquant qu'à la même concentration en poids sec, l'osmolalité d'une maltodextrine classique augmente avec l'abaissement de son DP.

De manière générale, les maltodextrines sont bien absorbées par le corps humain, mais dans des conditions physiques plus extrêmes, tel que l'exercice sportif ou la maladie, il faut assurer un meilleur apport en hydrates de carbone.

Par exemple, chez les sportifs, une boisson consommée pendant une activité physique qui requiert beaucoup d'effort se doit d'apporter instantanément l'énergie et l'eau nécessaire pour compenser la perte de fluide par perspiration.

Il résulte de ce qui a été énoncé plus haut qu'une composition équilibrée en hydrates de carbone est essentielle pour obtenir un tel résultat.

Une solution classiquement proposée pour la boisson optimale est de choisir de courts oligosaccharides linéaires de DP 3 à 6, puisqu'ils sont absorbés à la fréquence la plus élevée, tout en gardant l'osmolalité à un niveau modéré, empêchant ainsi la perte des fluides et les effets secondaires tels la diarrhée et les crampes.

Cependant, ces compositions présentent l'inconvénient de constituer des sources d'énergie trop instantanément assimilables par l'organisme, ce qui se traduit par des difficultés à maintenir un apport énergétique constant sur de longues périodes de temps.

Il a ainsi été proposé dans la demande de brevet WO 95/22.562 de nouveaux dérivés d'amidon destinés à la fourniture d'énergie pour la préparation ou après un effort physique.

Il s'agit de dextrines, caractérisées par leur poids moléculaire compris entre 15. 10³ et 10⁷ daltons, et un degré de branchement glucosidique 1,6 compris entre 2 et 8 %, préférentiellement compris entre 3 et 7 %, qui assurent un renouvellement des réserves énergétiques sous forme de glycogène.

Sous leur forme liquide, ces dextrines particulières passent dans l'intestin grêle après une vidange gastrique rapide. Cette voie est par ailleurs contrôlée par l'osmolalité desdites dextrines.

Une osmolalité élevée signifie ici que les substances de bas poids moléculaire se lient à l'eau, ce qui rend difficile le transport de l'eau et des nutriments dans la cellule. L'osmolalité du sang est d'environ 300 mOsm/l, et dans le but de faciliter le transport des nutriments, il est souhaitable que l'osmolalité de la substance soit notablement en dessous de cette valeur.

Une dextrine selon WO 95/22.562, d'un poids moléculaire moyen d'environ 720.000 et d'un degré de branchement de 4 % environ, est décrite comme présentant une osmolalité de 20 mOsm/kg sol.

Cependant, ces dextrines sont préparées par traitement acide de l'amidon natif, plus particulièrement de la fécule de pomme de terre, dans des conditions de température élevées, i.e. 110 à 140°C et dans un temps de réaction de 1 à 15 heures, ce qui conduit à un degré de branchement 1,6 qui correspond à la fois à des liaisons glucosidiques α-1,6 et β-1,6.

Ces liaisons glucosidiques atypiques ne sont pas digérées par les systèmes enzymatiques de l'intestin, et peuvent conduire à l'accumulation de résidus non digestibles que certaines bactéries indésirables vont assimiler.

Dans un autre domaine d'application, les maltodextrines sont souvent ajoutées aux boissons pour augmenter leur viscosité. Cependant, dans celles renfermant de l'alcool, l'apport de MD de haut DP peut générer des problèmes de stabilité du mélange.

Une autre solution qui consiste à ajouter du maltose ou du glucose conduit cependant à apporter une saveur sucrée additionnelle au mélange, ce qui n'est pas toujours souhaité. De plus, ces petits oligosaccharides peuvent servir de substrats de fermentation pour des microorganismes indésirables.

Les maltodextrines les plus adaptées à ce domaine d'application doivent donc concilier et équilibrer les paramètres de « non sucré », de viscosité et de stabilité.

Dans le domaine des solutions parentérales, des solutions nutritives sont conçues pour maintenir un patient en bonne santé et pour lui fournir les nutriments quand il ne peut être alimenté via son système digestif normal.

Puisque les solutions sont directement apportées par voie veineuse, elles se doivent d'être isotoniques et l'apport en glucose est limité.

Pour fournir une énergie journalière de 10.000 kJ, il est décrit dans un article de FOOD Science Technology de 1999, pp 345-355 par MARCHAL et al., qu'il serait nécessaire de perfuser 14 litres de solution isotonique de glucose (5 % poids/volume de glucose), ce qui dépasse largement les capacités humaines.

L'apport de solutions plus concentrées en glucose ou fructose (10 à 20 % poids/volume) est possible, mais pas pour de longues périodes.

Il est possible d'administrer des saccharides linéaires avec un DP compris entre 2 et 5, puisque ces saccharides sont hydrolysés par des maltases dans le rein, et que le glucose libéré est alors réabsorbé. C'est ainsi que l'utilisation de courts oligosaccharides linéaires permet d'apporter suffisamment d'énergie dans une solution isotonique, sans sur-hydrater le patient.

Par ailleurs, les oligosaccharides linéaires d'un DP inférieur à 7 étant stables en solution sur de longues périodes de temps, il est classiquement choisi de faire varier le DP entre 2 et 7 pour permettre d'apporter constamment aux patients, sur ces longues périodes, toute l'énergie nécessaire.

Mais cette solution n'est pas totalement satisfaisante, et elle n'envisage que l'exploitation de structures glucosidiques linéaires.

Quant à la nutrition entérale, elle concerne des boissons qui peuvent être ou bien ingérées oralement, ou administrées par voie tubulaire dans l'estomac ou l'intestin grêle.

Pour ces fluides entéraux, le problème majeur est la diarrhée, due à une trop forte osmolalité. En principe, la même solution que celle trouvée pour les sportifs peut également s'appliquer ici.

De manière classique, sont donc utilisées des maltodextrines renfermant un mélange complexe de saccharides linéaires et branchés, avec un DE de 10 à 20, mais sans donner toutefois entière satisfaction.

Les spécialistes de ces domaines d'applications recherchent la solution de ces problèmes techniques dans l'élaboration de structures ramifiées dérivés de l'amidon.

L'amylopectine, principal constituant de l'amidon, s'organise autour de liaisons α-1,4 linéaires et de liaisons α-1,6 qui s'y ramifient. La connaissance des microstructures a mis en évidence que ces deux types de liaisons ne se trouvent pas réparties de manière uniforme, mais que des zones très denses en liaisons α-1,6 y côtoient des zones constituées uniquement de liaisons α-1,4.

Il a été proposé, dans le brevet US 4.840.807, ou la demande de brevet JP 11/187.708, d'extraire les seules zones denses en liaisons α-1,6, comme source de glucides à absorption lente, au sens où les liaisons α-1,6 sont plus difficiles à dégrader que les liaisons α-1,4.

Deux familles de produits ont ainsi été développées. La première concerne les dextrines limites préparées par la dégradation des zones à liaisons α-1,4 par une α-amylase seule, et les dextrines préparées par la dégradation des zones à liaisons α-1,4 par l'action simultanée d'une α-amylase et d'une β-amylase.

La résistance de ces dextrines limites aux enzymes digestives humaines permet de les utiliser pour réguler la digestion, mais également pour contrôler la glycémie (application pour l'alimentation des diabétiques). Cet effet est attribué à un retard de la vitesse d'adsorption digestive.

Cependant, ces composés ont l'inconvénient de présenter un poids moléculaire très faible (compris entre 10.000 et 55.000 daltons), ce qui en limite l'exploitation dans d'autres domaines d'applications.

Le brevet EP 207.676 enseigne que pour un usage en dialyse péritonéale continue et ambulatoire, il est préféré des hydrolysats d'amidon formant des solutions limpides et incolores à 10 % dans l'eau, ayant un Mw de 5.10³ à 10⁶ daltons et un indice de polymolécularité ou Ip faible.

Cela se traduit par des compositions qui renferment majoritairement des polymères de glucose de haut poids moléculaire compris entre 5.10³ et 5. 10⁵ daltons), qui ne renferment pas ou très peu de glucose ou d'oligosaccharides de DP inférieur ou égal à 3, et pas ou très peu de polymères de glucose de Mw supérieur à 10⁶ daltons.

On conçoit en effet aisément pour cette application que les monomères ou polymères de faible poids moléculaire traversent rapidement la paroi péritonéale et sont ainsi sans intérêt durable pour la création d'un gradient de pression osmotique, et que les polymères de très haut poids moléculaire, dénués de pouvoir osmotique, sont à éviter et même à proscrire puisque potentiellement dangereux s'il leur advenait de précipiter consécutivement à leur rétrogradation.

La dialyse péritonéale consiste à introduire une solution de dialyse dans la cavité péritonéale au moyen d'un cathéter. Au bout d'un certain temps, un échange de solutés se produit entre le dialysat et le sang. L'utilisation d'un agent osmotique adéquat permet le drainage de l'eau excédentaire, du sang vers le dialysat.

La méthode standard en dialyse péritonéale pour éliminer l'excès d'eau (ultrafiltration) et de solutés de l'organisme en cas de déficience rénale consistait à utiliser une solution de dialyse rendue hypertonique par rapport au plasma par addition de glucose comme agent osmotique. Le flux à travers une membrane semi-perméable idéale est principalement déterminé par le nombre total de particules de soluté (osmolalité) présentes dans la solution, indépendamment de leur taille. En revanche, dans le cas d'une membrane biologique telle que la membrane péritonéale, le flux dépend uniquement des solutés ne traversant pas ou peu la membrane et n'est donc pas nécessairement lié à l'osmolalité totale de la solution. En outre, la capacité des solutés à traverser la membrane est caractérisée par la forme des molécules et leur charge ionique, ainsi que par leur taille.

Le choix d'un agent osmotique idéal est délicat : ce dernier doit permettre un gradient osmotique de manière à déplacer l'eau et les substances toxiques du sang vers la solution de dialyse à travers le péritoine. Il doit également être non toxique et biologiquement inerte, tout en étant métabolisable par l'organisme, une partie de celui-ci étant assimilée dans le sang. Il ne doit pas traverser la membrane péritonéale trop rapidement, de manière à maintenir durablement un gradient d'ultrafiltration sans accumuler de substances indésirables dans le sang.

Dans son brevet EP 667.356, la société Demanderesse a proposé un procédé de fabrication, à partir d'amidon waxy, d'un hydrolysat d'amidon complètement soluble dans l'eau et de faible indice de polymolécularité, inférieur à 2,8, ayant un Mw compris entre 5. 10³ et 1. 10⁶ daltons.

Ce procédé consiste à hydrolyser par voie acide un lait d'amidon constitué exclusivement d'amylopectine, puis à compléter cette hydrolyse acide par une hydrolyse enzymatique à l'aide d'α-amylase bactérienne, et de chromatographier sur résines cationiques fortes macroporeuses sous forme alcaline ou alcalino-terreuse.

Il est à noter qu'à l'époque, la société Demanderesse recommandait de n'utiliser que des amidons presque exclusivement composés d'amylopectine et couramment dénommés amidons waxy ou amidons cireux comme matière première dans ledit procédé, les amidons ou fécules contenant une proportion non négligeable d'amylose ne convenant pas.

Cet hydrolysat d'amidon, encore appelé icodextrine, a permis une diminution significative de l'absorption quotidienne de glucose préalablement utilisé comme agent osmotique dans les solutions pour dialyse, constituant ainsi un avantage potentiel pour le traitement des patients diabétiques et obèses pour lesquels la charge calorique est un facteur critique. Ceci pourrait toutefois être encore amélioré en utilisant un agent osmotique moins glycémiant, et dont le pouvoir osmotique durerait plus longtemps, ce qui permettrait d'alléger significativement la procédure du traitement de dialyse. En effet, le rendement des dialysats étant amélioré, la fréquence de changement des poches de dialyse serait diminuée, ce qui constitue une amélioration certaine de la qualité de vie du patient.

Ainsi, le glucide idéal en dialyse péritonéale devrait :
- être soluble dans l'eau
- exercer une pression osmotique
- avoir une faible viscosité
- ne pas rétrograder
- induire une cinétique d'apparition de glucose faible dans la circulation systémique
- être hydrolysé lentement par l'amylase de manière à exercer une pression osmotique durable.

En effet, en rapport avec ce dernier point, le devenir des agents osmotiques administrés en solution dans la cavité péritonéale chez des insuffisants rénaux est déterminé par sa stabilité dans le liquide péritonéal, l'importance de l'absorption dans la circulation systémique et la vitesse d'hydrolyse par l'amylase. Or les agents osmotiques de l'art antérieur présentent l'inconvénient d'être rapidement hydrolysés.
De même, les amidons dits résistants ont été proposés comme agents régulateurs de la glycémie. Or ceux-ci ne sont généralement pas stables dans les compositions, ne peuvent être stérilisés, ce qui engendre une perte de produit en final, et ceux-ci peuvent être fermentés et n'apportent donc pas la part calorique attendue.

De tout ce qui précède, il résulte qu'il y a donc un besoin non satisfait de disposer de polymères de glucose présentant des propriétés remarquables, notamment en terme de stabilité, de solubilité et éventuellement de viscosité, et conférant par-là même aux produits qui les contiennent des capacités plus grandes de durée de vie, de digestibilité contrôlée, ce qui en permet l'usage dans des domaines aussi variés que la dialyse péritonéale, la nutrition entérale ou parentérale, comme inhibiteur et/ou régulateur de la glycémie, comme apport énergétique lors d'activités physiques et comme régulateur de la digestion.

La Société Demanderesse a eu le mérite de concilier tous ces objectifs réputés jusqu'alors difficilement conciliables en imaginant et élaborant, au prix de nombreuses recherches, de nouveaux polymères solubles de glucose hautement branchés.

Les polymères solubles de glucose hautement branchés conformes à l'invention, ayant une teneur en sucres réducteurs inférieure à 11 %, sont ainsi caractérisés par le fait qu'ils possèdent un taux de liaisons glucosidiques α-1,6 compris entre 12 et 30 %, un Mw déterminé par diffusion de la lumière d'une valeur comprise entre 0,3. 10⁵ et 2. 10⁵ daltons, et une osmolalité, déterminée selon un test A, d'une valeur comprise entre 1 et 15 mOsm/kg.

Les polymères solubles de glucose branchés conformes à l'invention présentent une faible teneur en sucres réducteurs.

La détermination du pouvoir réducteur des polymères de glucose branchés conformes à l'invention, par toute méthode connue par ailleurs de l'homme du métier, conduit à des valeurs inférieures à 1 %.

Le taux de liaisons glucosidiques α-1,6 des polymères solubles de glucose branchés conformes à l'invention est déterminé par analyse RMN du proton. Le taux de branchement est alors exprimé en pour cent, correspondant à la quantité de signal du proton porté par le C1 d'un motif anhydroglucose qui lie un autre motif anhydroglucose par une liaison α-1,6, lorsque l'on a donné une valeur de 100 à l'ensemble des signaux des protons portés par tous les C1 des résidus glucose desdits polymères solubles de glucose.

Dans ces conditions, il est déterminé que les polymères de glucose solubles hautement branchés conformes à l'invention présentent un taux de liaisons α-1,6 qui est compris entre 12 % et 30 %.

Cette teneur en liaisons glucosidiques α-1,6 confère à tout polymère de glucose hautement branché conforme à l'invention une structure particulière, en termes de degré de ramification et/ou de longueurs de chaînes ramifiées par rapport à l'amidon ou au dérivé d'amidon dont il est issu.

Cette teneur particulièrement élevée en liaisons glucosidiques α-1,6 rend difficilement digestibles les polymères de glucose hautement branchés selon l'invention, ce qui contribue à pouvoir les utiliser comme agent régulateur de la digestion et comme agent inhibiteur de la glycémie, comme énoncé plus haut.

Ils peuvent donc être utilement proposés aux diabétiques ou aux sujets prédisposés, comme aliments, boissons ou adjuvants de nutrition ayant pour fonction d'inhiber l'élévation de la glycémie.

Les polymères solubles de glucose hautement branchés conformes à l'invention présentent également une absence de rétrogradation en solution aqueuse et une remarquable stabilité.

Cette propriété destine tout naturellement les polymères de glucose branchés conformes à l'invention à des compositions utilisables dans l'industrie alimentaire, qui présentent alors des stabilités élevées au stockage.

Un autre avantage de l'invention est de permettre l'obtention d'un produit fini utilisable par exemple comme liant instantané dans des produits réfrigérés ou surgelés.

La détermination des masses moléculaires des polymères solubles de glucose branchés conformes à l'invention est réalisée par la mesure des masses moléculaires moyennes en poids (Mw).

Cette valeur est obtenue par chromatographie d'exclusion stérique sur colonnes PSS SUPREMA 100 et PSS SUPREMA 1000 montées en série et couplées à un détecteur de diffusion de la lumière.

Les polymères de glucose branchés conformes à l'invention présentent alors une valeur de Mw comprise entre 0,3. 10⁵ et 2. 10⁵ daltons.

Les polymères de glucose solubles conformes à l'invention présentent également une osmolalité remarquablement faible.

Le test A consiste à déterminer l'osmolalité d'une solution renfermant 100 g sec de polymères de glucose hautement branchés conformes à l'invention placés dans 1 kg d'eau.

La mesure de l'osmolalité de cette solution est ensuite effectuée sur un osmomètre MARK 3 de FISKE® ASSOCIATES, en suivant les spécifications du constructeur.

Les polymères de glucose branchés conformes à l'invention présentent alors une valeur d'osmolalité remarquablement basse comprise entre 1 et 15 mOsm/kg.

Il n'existe pas, à la connaissance de la Société Demanderesse, de polymères de glucose qui possèdent une telle valeur d'osmolalité, pour des produits présentant par ailleurs un taux de branchement et un poids moléculaire tels que définis.

En effet, des mesures comparatives effectuées sur des maltodextrines classiques présentant un équivalent dextrose (DE) compris entre 5 et 20 montrent des valeurs d'osmolalité comprise entre 25 à 85 mOsm/kg.

D'autres mesures, effectuées sur des dextrines limites telles que définies ci-avant par traitement d'amidon liquéfié à l'α-amylase, commercialisées sous le nom BLD 8 par SANMATSU, présentent pour un poids moléculaire compris entre 0,4 et 0,5. 10⁵ daltons et un taux de branchement α-1,6 compris entre 8 et 9 %, une valeur d'osmolalité de plus de 35 mOsm/kg.

Cette valeur d'osmolalité très basse confère ainsi aux polymères solubles hautement branchés conformes à l'invention des propriétés qui leur permettent d'être mis en oeuvre dans des préparations destinées aux sportifs, pour renouveler les sources d'énergie dont ils ont besoin pour des efforts physiques sur de longues périodes.

Mais également et surtout, ces compositions peuvent être avantageusement utilisées pour des patients qui ne peuvent plus s'alimenter normalement, dans le cadre de nutrition entérale et parentérale.

Par ailleurs, associés à cette propriété de faible osmolalité, leur absence de rétrogradation, leur profil de poids moléculaire et leur faible indice de polydispersité font de ces polymères de glucose hautement branchés conformes à l'invention de parfaits candidats comme agents osmotiques pour des applications en dialyse péritonéale, comme il sera exemplifié ci-après. La Demanderesse a par ailleurs démontré que ces polymères conformes à l'invention présentent une résistance à l'alpha amylase qui apporte des avantages significatifs par rapport aux polymères de l'art antérieur, pour un poids moléculaire similaire, puisqu'ils sont moins glycémiants et présentent un pouvoir osmotique qui dure plus longtemps, autorisant ainsi leur usage dans les traitements de dialyse de longue durée.

De manière avantageuse, les polymères de glucose hautement branchés conformes à l'invention peuvent être classés en trois sous-familles en fonction de leur osmolalité.

La première sous-famille couvre des polymères hautement branchés qui présentent, pour un Mw déterminé par diffusion de la lumière d'une valeur comprise entre 0,5. 10⁵ et 1,5. 10⁵ daltons, une osmolalité, déterminée selon le test A, au moins égale à 1 et inférieure à 2 mOsm/kg.

La deuxième sous-famille couvre des polymères hautement branchés qui présentent, pour un Mw déterminé par diffusion de la lumière d'une valeur comprise entre 0,5. 10⁵ et 0,8. 10⁵ daltons, une osmolalité, déterminée selon le test A, au moins égale à 2 et inférieure à 5 mOsm/kg.

La Société Demanderesse a en outre trouvé des polymères de glucose branchés appartenant à ces deux sous familles présentant de plus un taux de branchement α-1/6 remarquablement élevé, i.e. compris entre 15 et 30 %.

La troisième sous-famille couvre des polymères hautement branchés qui présentent un Mw déterminé par diffusion de la lumière compris entre 0,3. 10⁵ et 0,7. 10⁵ daltons et une osmolalité, déterminée selon le test A, au moins égale à 5 et inférieure à 15 mOsm/kg.

Pour préparer les polymères solubles de glucose branchés conformes à l'invention, on réalise la succession des étapes suivantes consistant à :
a. préparer une suspension aqueuse d'amidon ou une solution de dérivé d'amidon d'une matière sèche au moins égale 1 % en poids, de préférence de 10 à 50 % en poids,
b. traiter ladite suspension ou ladite solution avec au moins une enzyme de branchement à une température comprise entre 25 et 80°C pendant une durée de 1 à 24 heures,
c. faire agir sur la suspension ou sur la solution ainsi obtenue au moins une enzyme choisie dans le groupe constituée de la β-amylase, l'amyloglucosidase et l'α-transglucosidase,
d. effectuer un fractionnement à l'aide d'au moins une technique choisie dans le groupe des séparations sur membrane ou des chromatographies, de manière à récupérer les fractions de haut poids moléculaire,
e. recueillir les polymères de glucose branchés ainsi obtenus.

L'amidon est introduit en suspension, ou les dérivés d'amidon en solution aqueuse, à une matière sèche au moins égale 1 % en poids, de préférence de 10 à 50 % en poids.

Le choix d'une origine, ou d'une qualité d'amidon ou de ses dérivés particuliers, ne revêt qu'une importance relative.

De fait, la société Demanderesse a mis au point un nouveau procédé, permettant d'obtenir les polymères de glucose hautement branchés conformes à l'invention, par exemple applicables en dialyse péritonéale, qui ne nécessite pas de se limiter à un type particulier d'amidon, en l'occurrence un amidon riche en amylopectine.

Il peut donc être choisi l'amidon naturel ou hybride issu de pomme de terre, de pomme de terre à haute teneur en amylopectine (fécule waxy), de pois, de riz, de manioc, de blé, de maïs, de maïs ou de blé riches en amylopectine (maïs ou blé waxy), de maïs à haute teneur en amylose, de coupes ou de fractions qui peuvent être faites ou obtenues à partir d'amidons, telles que l'amylose, l'amylopectine, les coupes granulométriques connues de l'homme de l'art sous les vocables d'amidon de blé « A » et amidon de blé « B », et les mélanges d'au moins deux quelconques des produits susmentionnés.

Les dérivés d'amidon peuvent s'entendre des amidons modifiés issus de la modification enzymatique, chimique et/ou physique, en une ou plusieurs étapes, de cet amidon.

Les dérivés d'amidon peuvent notamment être les amidons modifiés par l'une au moins des techniques connues d'éthérification, d'estérification, de réticulation, d'oxydation, de traitement alcalin, d'hydrolyse acide et/ou enzymatique (à l'origine des maltodextrines et dextrines).

La Société Demanderesse a trouvé que les polymères de glucose hautement branchés conformes à l'invention sont aisément synthétisables à partir d'amidons, ou de leurs dérivés, qui présentent déjà un taux de branchement au moins égal à 1 %.

Cette suspension d'amidon, ou cette solution de dérivés d'amidon, peut être éventuellement soumise ensuite à un traitement par cuisson particulier, qui consiste à la traiter à une température supérieure à 130°C, de préférence comprise entre 140 et 150°C, sous une pression de plus de 3,5 bars, de préférence comprise entre 4 à 5 bars, pendant 30 secondes à 15 minutes, de préférence pendant 1 à 5 minutes.

Ce traitement est avantageusement réalisé dans un cuiseur tubulaire à double enveloppe chauffé par fluide thermique, équipement qu'il est aisé à l'homme du métier de se procurer.

La deuxième étape du procédé conforme à l'invention consiste à traiter ladite suspension d'amidon ou ladite solution de dérivé d'amidon avec une enzyme de branchement.

De façon avantageuse, on utilise de 50.000 à 500.000 U d'enzyme de branchement purifiée pour 100 g sec d'amidon ou de dérivé d'amidon, à une température comprise entre 25 et 95°C, de préférence à une température comprise entre 70 et 95°C, pendant une durée de 1 à 24 heures.

Par enzymes de branchement, on entend au sens de l'invention les enzymes de branchement choisies dans le groupe constitué par les enzymes de branchement du glycogène, les enzymes de branchement de l'amidon et les mélanges quelconques de ces enzymes.

Plus particulièrement, ces enzymes de branchement sont extraites d'organismes et/ou de micro-organismes choisis dans le groupe constitué par les enzymes de branchement du glycogène, les enzymes de branchement de l'amidon et les mélanges quelconques de ces enzymes.

La Société Demanderesse préfère, pour effectuer ce traitement avec une enzyme de branchement, suivre l'enseignement de sa demande de brevet WO 00/18.893.

Cette étape conduit à produire des polymères de glucose solubles branchés, mais avec une teneur en liaisons glucosidiques α-1,6 au mieux égale à 10 %.

Pour augmenter cette valeur et atteindre des taux de liaisons α-1,6 jusqu'à 30 %, la société Demanderesse a trouvé qu'il faut réaliser un traitement enzymatique complémentaire, et c'est ce qui constitue la troisième étape du procédé d'obtention des polymères de glucose solubles hautement branchés conformes à l'invention.

Cette troisième étape consiste à faire agir sur la suspension ou la solution traitée avec une enzyme de branchement ainsi obtenue, au moins une enzyme choisie dans le groupe constitué de la β-amylase, l'amyloglucosidase et l'α-transglucosidase,

Les conditions d'action (température et pH) des différentes enzymes mises en oeuvre dans le procédé conforme à l'invention sont choisies parmi les suivantes (les quantités sont fixées en regard du substrat considéré, comme il sera exemplifié ci-après):
- α-amylase : de type LYSASE 2000 de GENENCOR, à une température de 55 à 65°C, pH de 6,5 à 6,7, pendant 30 minutes à 1 heure (comparatif).
- β-amylase : de type SPEZYME BBA de GENENCOR, à une température de 40°C, pH de 4,9 à 5, pendant 1 h 30 à 2 heures.
- amyloglucosidase : soit de type OPTIDEX L300 A de GENENCOR à une température de 55°C, pH de 4,7 ; soit de type A-7420 de SIGMA à une température de 50°C à 60°C, pH de 4,7 à 4,9 ; pendant 1 h 30 à 2 heures.
- α-transglucosidase : de type α-TGase de L-AMANO à une température de 55°C, pH de 5 à 5,2, pendant 1 heure.

Les enzymes utilisées peuvent être d'origine bactérienne ou fongique.

Au terme de ce traitement complémentaire, les polymères de glucose hautement branchés solubles sont obtenus en mélange avec leurs produits de dégradation enzymatiques, majoritairement constitués de glucose, de maltose et/ou d'isomaltose, comme il sera exemplifié ci-après.

La quatrième étape du procédé consiste à effectuer un fractionnement à l'aide d'une technique choisie dans le groupe des séparations sur membrane et des chromatographies, de manière à récupérer les fractions de haut poids moléculaire et les fractions de bas poids moléculaire.

Les fractions de haut poids moléculaire correspondent aux polymères de glucose hautement branchés conformes à l'invention, tandis que les fractions de bas poids moléculaire permettent d'obtenir, avec un excellent rendement, des compositions enrichies en maltose et/ou isomaltose.

De manière avantageuse, on choisit une technique de fractionnement dans le groupe constitué par la technique de séparation sur membrane d'ultrafiltration et par la technique de séparation chromatographique sur support de type gel.

Dans un premier mode de réalisation de cette quatrième étape du procédé, le fractionnement est conduit à l'aide d'une technique de séparation sur membrane d'ultrafiltration, en utilisant une membrane ayant un seuil de coupure au moins égal à 3000 daltons, de préférence au moins égal à 5000 daltons.

Les fractions de haut poids moléculaire correspondant aux polymères de glucose hautement branchés, égales au rétentat de l'ultrafiltrat représentent alors environ 60 % de la matière sèche mise en oeuvre.

Dans un second mode de réalisation de cette quatrième étape du procédé, le fractionnement est conduit à l'aide d'une technique de chromatographie effectuée sur une résine de type gel.

Les profils obtenus permettent la séparation des fractions renfermant les polymères de glucose hautement branchés avec un rendement optimal compris entre 40 et 45 %.

Quelle que soit la méthode mise en oeuvre, les profils obtenus permettent la séparation de la fraction polysaccharidique de haut poids moléculaire correspondant aux polymères de glucose branchés solubles conformes à l'invention, des fractions oligosaccharidiques de bas poids moléculaire, constituées pour l'essentiel de glucose et de maltose et/ou d'isomaltose.

La dernière étape du procédé conforme à l'invention consiste donc à collecter d'une part les fractions de haut poids moléculaire correspondant aux polymères de glucose hautement branchés, et d'autre part les fractions de bas poids moléculaire enrichies en glucose et isomaltose et/ou en maltose.

Les produits de haut poids moléculaire peuvent être rassemblés en tant que tels, ou précipités par 3 volumes d'éthanol, purifiés et séchés sous vide pendant 24 heures, ou encore atomisés, par toute technique connue de l'homme du métier.

Quant aux compositions enrichies en maltose et/ou isomaltose, caractérisées en ce qu'elles comprennent les fractions de bas poids moléculaire de l'étape d du procédé conforme à l'invention, elles pourront être utilisées en tant que telles, ou hydrogénées par toute technique d'hydrogénation connues par ailleurs de l'homme du métier.

Les caractéristiques physico-chimiques particulières des polymères selon l'invention permettent leurs applications dans les industries notamment du Papier-Carton, du Textile, de la Cosmétique, et en particulier de la Pharmacie et de l'Alimentaire, et plus particulièrement encore dans les domaines de la nutrition entérale et parentérale, de la dialyse péritonéale en tant qu'agent osmotique, comme agent inhibiteur de la glycémie, comme apport énergétique lors d'activités physiques et comme agent régulateur de la digestion.

En ce qui le domaine particulier de la dialyse péritonéale, la Demanderesse a trouvé, à l'aide d'un test de résistance à l'alpha-amylase, qu'une famille de polymères conformes à l'invention était particulièrement adaptée à la préparation de solutions pour dialyse péritonéale, comme il le sera exemplifié par la suite. Ces polymères y sont utilisés comme agent osmotique.

L'invention a donc pour objet une solution pour dialyse péritonéale caractérisée en ce qu'elle comprend en tant qu'agent osmotique au moins un polymère soluble hautement branché conforme à l'invention.

Selon une variante préférée de l'invention, ledit polymère présente :
- un Mw, déterminé par diffusion de la lumière d'une valeur comprise entre 0,3. 10⁵ et 0,7. 10⁵ daltons,
- une osmolalité, déterminée selon un test A, au moins égale à 5 et inférieure à 15 mOsm/kg.

La solution pour dialyse péritonéale selon l'invention peut en outre comprendre des électrolytes physiologiquement acceptables, comme le sodium, le potassium, le calcium, le magnésium, le chlore, de manière à éviter la perte par transfert d'électrolytes du sérum vers le péritoine.

Cette solution peut se présenter par exemple en solution aqueuse. Dans ce dernier cas, la solution obtenue par dissolution dans l'eau des polymères hautement branchés selon l'invention doit être limpide et incolore. Cette solution doit être de préférence exempte d'endotoxines, de peptido-glucans et de bêta-glucans, ainsi que de contaminants azotés provenant de la matière première, ou des préparations enzymatiques utilisées pour sa fabrication.

A cet effet, les polymères hautement branchés mis en oeuvre dans ladite solution auront de préférence subi une purification de manière à ôter toute coloration ou tout contaminant indésirable tel que protéines, bactéries, toxines bactériennes, fibres, traces de métaux etc...

Cette étape de purification peut être réalisée selon les techniques connues de l'homme du métier.

La solution pour dialyse selon l'invention peut comprendre également des solutions tampons (lactate, acétate, gluconate notamment) et autres additifs tels que des amino-acides, de l'insuline, des polyols tels que par exemple le sorbitol, l'érythritol le mannitol, le maltitol, le xylitol.

L'ajout de polyols à la composition, et de préférence de polyols apyrogènes et exempts des impuretés décrites précédemment (endotoxines et autres résidus d'origine bactérienne notamment) permet d'augmenter l'osmolarité de la solution de manière plus avantageuse que le glucose ou le maltose, en raison de leur plus faible caloricité, de leur pouvoir osmotique supérieur et parce qu'ils ne sont pas réducteurs.

La composition pour dialyse selon l'invention est avantageuse par rapport aux produits de l'art antérieur puisque l'agent osmotique qu'elle contient permet d'exercer une pression osmotique durable et induit une cinétique d'apparition de glucose faible, tout en étant stable à la rétrogradation, répondant ainsi aux principaux critères définis précédemment.

D'autres caractéristiques et avantages de l'invention apparaîtront à la lecture des exemples non limitatifs décrits ci-dessous.

### Exemple 1

On prépare une solution de dérivés d'amidon d'une teneur en matière sèche de 25 % en poids, par chauffage à 80°C sous agitation lente et continue.

Il s'agit en l'occurrence ici de deux maltodextrines commercialisées par la société Demanderesse sous le nom de GLUCIDEX® 2 (substrat A) et GLUCIDEX® 6 (substrat B) à 250 g/l.

Cette solution est refroidie à 30°C, et le pH est ramené à 6,8 par NaOH 1N.

On traite ensuite ces solutions par de l'enzyme de branchement du glycogène purifiée, extraite du microorganisme *B. stearothermophilus.*

On ajoute l'enzyme de branchement à raison de 1600 U/g de substrat, et amène progressivement la température à 65°C.

L'incubation est réalisée sous agitation modérée pendant 4 heures. La réaction est ensuite stoppée par abaissement du pH à une valeur de 5 et par ébullition pendant 6 minutes.

Le tableau I ci-après rassemble, pour les deux substrats testés, les résultats obtenus en termes de teneurs en liaisons glucosidiques α-1,6, de valeurs des Mw, de teneurs en sucres réducteurs et d'osmolalité pour les produits obtenus (produit C à partir du substrat A et produit D à partir du substrat B).

**Tableau I**

| | % de liaisons α-1,6 | Mw 10⁵ daltons | % en sucres réducteurs | Osmolalité mOsm/kg |
|---|---|---|---|---|
| A | 5,9 | 4,88 | 2 | 16 |
| C | 8,7 | 1,19 | 1,5 | 12 |
| B | 5,4 | 0,9 | 3,8 | 25 |
| D | 8 | 0,61 | 4,3 | 25 |

La teneur en liaisons glucosidiques α-1,6 est sensiblement augmentée, mais n'atteint pas encore les valeurs souhaitées.

La Société Demanderesse a donc trouvé qu'un traitement complémentaire devait être mené, par l'action d'enzymes hydrolysant spécifiquement les liaisons glucosidiques α-1,4 (telles la β-amylase ou l'amyloglucosidase), ou par l'utilisation d'enzymes qui complètent le branchement en liaisons α-1,6 (telle l'α-transglucosidase), et ce, de la manière suivante.

Pour les traitements enzymatiques complémentaires, les solutions des maltodextrines branchées C et D sont tout d'abord amenées à la température et au pH de l'enzyme choisie.
1) Pour le traitement complémentaire à l'α-amylase (LYSASE 2000 à 2444 UBR/g d'extrait enzymatique), lesdites solutions de C ou de D sont amenées à la température de 60°C et au pH de 6,5 à 6,7, et on ajoute 6 U d'α-amylase par g de substrat.
   L'incubation est réalisée pendant 30 minutes, et la réaction est stoppée par ébullition 6 minutes.
2) Pour le traitement complémentaire à la β-amylase (BBA SPEZYME de GENENCOR), lesdites solutions de C ou de D sont amenées à la température de 40°C et au pH de 4,9 à 5, et on ajoute 30 U de β-amylase par g de substrat.
   L'incubation est réalisée pendant 2 heures, et la réaction est stoppée par ébullition 6 minutes.
3) Pour le traitement complémentaire à l'amyloglucosidase (AMG de SIGMA AA-7420 d'*A. niger,* à 40 U/mg de protéines), lesdites solutions de C ou de D sont amenées à la température de 55°C et au pH de 4,7 à 4,9, et on ajoute 20 U de l'AMG par g de substrat.
   L'incubation est réalisée sous agitation modérée pendant 2 heures, et la réaction est stoppée par ébullition 6 minutes.
4) Pour le traitement complémentaire à l'α-transglucosidase (α-TGase L-AMANO, activité de 27,7 µmoles en glucose), lesdites solutions de C ou de D sont amenées à la température de 55°C et au pH de 5 à 5,2, et on ajoute 2 U de l'α-TGase par g de substrat.

L'incubation est réalisée pendant 1 heure, et la réaction est stoppée par ébullition 6 minutes.

On détermine ensuite les caractéristiques physico-chimiques :
- des produits E et F (obtenus par traitement complémentaire à l'α-amylase des produits respectivement C et D) ,
- des produits G et H (obtenus par traitement complémentaire à la β-amylase des produits respectivement C et D) ,
- des produits I et J (obtenus par traitement complémentaire à l'AMG des produits respectivement C et D) et
- des produits K et L (obtenus par traitement complémentaire à l'α-TGase des produits respectivement C et D)

**Tableau II**

| | % de liaisons α-1,6 | Mw 10⁵ daltons | % en sucres réducteurs | Osmolalité mOsm/kg |
|---|---|---|---|---|
| E* | 8,3 | 0,72 | 3,8 | 29 |
| G | 8,4 | 0,76 | 22,5 | 132 |
| I | 9 | 0,49 | 55 | 320 |
| K | 9,6 | 1,2 | 22 | 153 |
| F* | 7,4 | 0,44 | 8,7 | 52 |
| H | 7,2 | 0,51 | 23,8 | 141 |
| J | 7,8 | 0,47 | 50,5 | 301 |
| L | 12 | 0,69 | 28 | 192 |

| | | | | |
|---|---|---|---|---|
| * exemples comparatifs | | | | |

L'osmolalité et la teneur en sucres réducteurs qui augmentent, traduisent ici la production concomitante principalement de glucose, de DP2(maltose et isomaltose), qu'il est donc nécessaire d'éliminer pour obtenir les polymères de glucose hautement branchés conformes à l'invention.

Il est choisi d'utiliser un fractionnement par ultrafiltration sur membrane avec un seuil de coupure de 5000 daltons (membrane 5K AMICON).

Les résultats obtenus pour les produits d'ultrafiltration M, O, Q, S des composés respectivement E, G, I et K d'une part (donc issus de la GLUCIDEX® 2), et des produits d'ultrafiltration N, P, R, T des composés respectivement F, H, J et L (donc issus de la GLUCIDEX® 6) , sont présentés dans le tableau III suivant.

**Tableau III**

| | % de liaisons α-1,6 | Mw 10⁵ daltons | % en sucres réducteurs | Osmolalité mOsm/kg |
|---|---|---|---|---|
| M* | 10,2 | 0,89 | 0,42 | 1 |
| O | 15 | 0,75 | 0,3 | 1 |
| Q | 18,8 | 0,57 | 0,37 | 2 |
| S | 12,1 | 1,22 | 0,33 | 1 |
| N* | 10,9 | 0,69 | 0,54 | 2 |
| P | 14,4 | 0,51 | 0,8 | 3 |
| R | 18,1 | 0,55 | 0,5 | 5 |
| T | 12,2 | 0,72 | 0,6 | 3 |

| | | | | |
|---|---|---|---|---|
| * Exemples comparatifs | | | | |

Ces résultats traduisent le fait que les polymères de glucose hautement branchés ainsi obtenus présentent le parfait équilibre entre taux de liaisons glucosidiques α-1,6 remarquablement élevé (jusqu'à 18%), pour des produits qui présentent une telle valeur de Mw et une valeur aussi basse d'osmolalité.

Ces polymères de glucose hautement branchés peuvent être aisément mélangés avec d'autres électrolytes pour fournir des agents osmotiques extrêmement performants en dialyse péritonéale, ou être utilisés en tant que tels dans des compositions destinées à réguler la digestion, pour la nutrition parentérale et entérale, pour des compositions destinées aux diabétiques, ou encore en boissons liquides pour reconstituer les réserves d'énergie pour sportifs durant un effort physique de longue durée.

Il est à noter qu'outre ces polymères de glucose hautement branchés, le procédé permet également de rassembler les fractions enrichies en maltose et/ou isomaltose.

Par exemple, dans le cas de la préparation des produits S et T (issus du traitement combiné avec l'enzyme de branchement et avec l'α-TGase), l'isomaltose et le glucose sont les seuls co-produits fabriqués (aux concentrations respectives de 25 à 30 g/l et 75 à 80 g/l.

De la même façon, dans le cas de la préparation des produits O et P (issus du traitement combiné avec l'enzyme de branchement et la β-amylase), le maltose est le seul coproduit fabriqué (à la concentration de 130 g/l).

Ces fractions de bas poids moléculaire peuvent donc constituer des sources avantageuses de compositions riches en maltose et/ou isomaltose.

### Exemple 2

Les polymères de glucose hautement branchés conformes à l'invention peuvent être également préparés à partir d'amidon de maïs standard. Pour cela, on remet en suspension 110 g sec d'amidon dans un litre d'eau à température ambiante et sous agitation lente et continue.

On amène le pH de 6,8 à 7 et laisse dans ces conditions pendant 15 minutes, en rectifiant le pH si nécessaire. On ajoute l'enzyme de branchement du glycogène purifiée de *B. stearothermophilus* à raison de 4000 U/g de substrat, en amenant progressivement la température à 72 à 75°C.

L'incubation se fait ensuite sous agitation modérée pendant 30 minutes, puis refroidissement à une température de 65 à 68°C. La réaction enzymatique est menée pendant 4 heures. La réaction est ensuite stoppée par abaissement du pH à une valeur de 4,5 à 5, puis portée à ébullition pendant 6 minutes.

Comme dans l'exemple 1, la réaction est complétée par traitements à la β-amylase ou à l'amyloglucosidase, puis par une étape d'ultrafiltration sur membrane d'un seuil de coupure de 5000 daltons dans les conditions données dans l'exemple 1.

Le tableau IV rassemble les résultats obtenus.

L'amidon de maïs standard est référencé U ; le produit de traitement à l'enzyme de branchement V, ceux traités en complément par la β-amylase : W, par l'AMG : X ; Les produits finaux ultrafiltrés : Y et Z.

**Tableau IV**

| | % de liaisons α-1,6 | Mw 10⁵ daltons | % en sucres réducteurs | Osmolalité mOsm/kg |
|---|---|---|---|---|
| U | 3,6 | 110 | < 0,01 | Nd |
| V | 8,8 | 1,75 | 0,2 | 2 |
| W | 8,9 | 1,31 | 20,5 | 117 |
| Y | 16,3 | 1,38 | 0,1 | 2 |
| X | 7,9 | 0,55 | 55 | 357 |
| Z | 24,2 | 0,45 | 0,4 | 2 |

Les produits Y et Z obtenus présentent les mêmes profils équilibrés que ceux décrits à l'exemple 1, et donc peuvent être avantageusement mis en oeuvre dans les mêmes domaines d'applications.

### Exemple 3

Deux autres polymères de glucose hautement branchés sont préparés à partir de deux variétés d'amidon riche en amylopectine, dans des conditions industrielles. Il s'agit de deux échantillons d'amidon de maïs waxy fluidifié acide avec un niveau de fluidification WF d'environ 90, commercialisés également par la Société Demanderesse sous le nom de marque CLEARGUM® CB 90.

Le tableau V présente les conditions opératoires mises en oeuvre pour parvenir aux polymères de glucose hautement branchés conformes à l'invention.

**Tableau V**

| **Base** | CLEARGUM CB90 | CLEARGUM CB90 |
|---|---|---|
| **Solubilisation** | Cuiseur continu labo à 25% MS | Cuiseur continu labo à 25% MS |
| **1^{ère} Enzyme** | Enzyme de branchement 50.000 U/ml - | Enzyme de branchement 50.000 U/ml - |
| | 1ml / 100gsec | 1ml / 100gsec |
| **1^{er} traitement enzymatique** | 70°C pH 6,8 | 70°C pH 6,8 |
| | 22 h puis désactivation | 22 h puis désactivation |
| | 1 h à 90-95°C | 1 h à 90-95°C |
| **2^{ème} Enzyme** | Amyloglucosidase OPTIDEX L300A | β-amylase |
| | 0,08ml / 100gsec + 0,08ml / 100g sec | SPEZYME BBA |
| | après 1h30 | 0,2ml / 100g sec |
| **2^{ème} traitement enzymatique** | 55°C pH4,7 - 2 h et 3 h puis désactivation 1h à 90-95°C | 40°C pH 5 2 h puis désactivation 1 h à 90-95°C |
| **Purification** | Filtration sur 8 puis 0,22 µm - Ultrafiltration sur membranes PCI Membrane Systems ES209 (9000Da) - Traitement Noir NORIT SX+ 5% c/s pH5 70°C 1h - Ajustement du pH à 5,8 et filtration sur 8 puis 0,22 µm | Ajustement du pH à 3,5 durant 18h - Centrifugation 5000 tr/min 10min - Filtration sur terre filtrante puis 8 µm - Ultrafiltration sur membranes PCI Membrane Systems ES209 (9000Da) - Traitement Noir NORIT SX+ 5% c/s pH5 70°C 1h - Filtration sur 8 puis 0,22µm |
| **Récupération** | Concentration à sec au rotavapor sous vide | Concentration à sec au rotavapor sous vide |

Le tableau VI présente les résultats obtenus en termes de teneurs en liaisons glucosidiques α-1,6, de valeurs des Mw, de teneurs en sucres réducteurs et d'osmolalité pour les produits obtenus :
- « a » concerne le produit issu de CLEARGUM® CB 90, après traitement avec l'enzyme de branchement et l'amyloglucosidase et
- « b » concerne le produit issu de CLEARGUM® CB 90, après traitement avec l'enzyme de branchement et la β-amylase.

**Tableau VI**

| | % de liaisons α-1,6 | Mp 10⁵ daltons | % en sucres réducteurs | Osmolalité mOsm/kg |
|---|---|---|---|---|
| « a » | 19,4 | 0,33 | 1 | 12 |
| « b » | 14,3 | 0,68 | 0,7 | 6 |

Ces résultats montrent bien que le procédé mis en oeuvre permet d'obtenir des polymères de glucose hautement branchés conformes à l'invention quelle que soit la base amidon ou dérivé d'amidon choisie.

### Exemple 4

On prépare des solutions aqueuses de polymères hautement branchés conformes à l'invention, que l'on met en contact d'une amylase d'origine pancréatique. L'hydrolyse amylasique est suivie dans le temps par mesure des sucres réducteurs formés et par mesure du glucose apparaissant dans le milieu réactionnel. Ce test permet d'évaluer la résistance des polymères à l'hydrolyse amylasique, qui est un critère essentiel dans le choix d'un agent osmotique pour solution de dialyse.

Plusieurs polymères conformes à l'invention sont testés en comparaison avec l'icodextrine (agent osmotique de l'art antérieur). Les polymères sont choisis de manière à présenter un poids moléculaire voisin de cette dernière :
Produits A et B tels que préparés conformément à l'exemple 3 et produit Z tel que préparé conformément à l'exempl 2.
L'icodextrine est fabriquée conformément au brevet EP 667.356 cité dans la description.
Un témoin maltose est réalisé pour valider le modèle in vitro de digestion enzymatique.
Les conditions opératoires pour la digestion amylasique sont les suivantes :
   - Peser environ 0,6 g de produit à tester précisément,
   - Ajouter 150 mL de tampon maléate de Na pH 7 à 0,1mol /L,
   - Agiter jusqu'à la dissolution du produit,
   - Prélever 1,5 mL de la solution obtenue (solution initiale = si),
   - Placer les flacons au bain-marie pendant 15 minutes, pour que la température de la solution soit de 37°C,
   - Ajouter 0,15 g de pancréatine de porc (α-amylase d'origine animale),
   - Incuber à 37°C au bain thermostaté sous agitation pendant 300 minutes fTA,
   - Réaliser des prélèvements de 1,5 mL aux temps : 15, 30, 45, 60, 90, 120, 180, 240, 300 minutes,
   - Arrêter la réaction enzymatique en plaçant les prélèvements dans un bain à sec à 100°C, pendant 10 minutes,
   - Doser le glucose sur les prélèvements, pour simuler l'impact sur la glycémie du produit étudié,
   - Doser les sucres réducteurs sur les prélèvements pour étudier la vitesse d'hydrolyse.

Pour le dosage du glucose, on utilise une méthode colorimétrique, réalisée sur automate HITACHI 704 (ROCHE). Le réactif utilisé est un réactif contenant les enzymes GOD/PAP (glucose oxydase, péroxydase). Le volume de réactif utilisé est de 500 microlitres, le volume d'échantillon est de 5 microlitres et la température de la réaction est de 30°C.

La méthode utilisée pour le dosage des sucres réducteurs est la méthode de SOMOGYI NELSON. Dans un tube bouché, on introduit 200 microlitres d'échantillon, on ajoute 200 microlitres de solution de travail (réactifs tartrate de sodium et sulfate de cuivre). On porte à ébullition, on ajoute après refroidissement du réactif arsénomolybdique, puis de l'eau. La solution obtenue est déposée dans une microplaque, puis on lit l'absorbance au lecteur de microplaques à une longueur d'onde de 520 nanomètres.

Les résultats sont consignés dans les tableaux suivants :
1. cinétique d'apparition du glucose (en % libéré sur sec)

| **Temps (en min)** | **MALTOSE** | **A** | **B** | **Z** | **ICODEXTRINE** |
|---|---|---|---|---|---|
| si | 0,26 | 3,35 | 0,00 | 0,53 | 0,28 |
| 0 | 0,26 | 4,75 | 0,93 | 1,19 | 1,96 |
| 15 | 0,79 | 5,31 | 1,59 | - | 3,07 |
| 30 | 1,06 | 5,68 | 1,96 | 2,11 | 3,63 |
| 45 | 1,59 | 5,86 | 2,24 | - | 3,91 |
| 60 | 2,12 | 6,14 | 2,52 | 2,37 | 4,19 |
| 90 | 2,65 | 6,52 | 2,89 | - | 4,75 |
| 120 | 3,44 | 6,61 | 3,17 | 2,90 | 5,31 |
| 180 | 5,03 | 7,26 | 4,76 | 3,43 | 6,15 |
| 240 | 6,35 | 8,10 | - | 3,96 | 6,99 |
| 300 | 7,68 | 8,38 | 5,41 | 4,22 | 7,82 |

2. cinétique d'apparition des sucres réducteurs (en % sur sec)

| **Temps (en min)** | **MALTOSE** | **A** | **B** | **Z** | **ICODEXTRINE** |
|---|---|---|---|---|---|
| si | 51,01 | 5,76 | 0,88 | 1,45 | 2,74 |
| 0 | 49,82 | 18,34 | 18,04 | 8,92 | 31,07 |
| 15 | 47,94 | 19,33 | 18,96 | - | 30,39 |
| 30 | 48,29 | 20,00 | 19,04 | 11,00 | 32,53 |
| 45 | 48,55 | 20,25 | 19,78 | - | 32,46 |
| 60 | 48,84 | 19,92 | 20,80 | 11,10 | 32,95 |
| 90 | 49,42 | 20,37 | 19,42 | - | 34,16 |
| 120 | 47,15 | 21,68 | 21,04 | 12,16 | 34,40 |
| 180 | 48,87 | 22,46 | 21,79 | 12,22 | 36,64 |
| 240 | 50,90 | 23,05 | 23,11 | 12,29 | 37,03 |
| 300 | 52,20 | 22,67 | 22,88 | 13,64 | 37,06 |

3. synthèse des résultats

| PRODUITS TESTES | % de glucose libéré à 300 min. | % de sucres réducteurs formés à 300 min. | Taux de liaisons α-1,6 en % | Masse Molaire (daltons) |
|---|---|---|---|---|
| MALTOSE | 7,41 | 52,20 0 | 0 | 342 |
| A | 5,03 | 16,91 | 19,4 | 33000 |
| B | 5,41 | 22,88 | 14,3 | 68000 |
| Z | 3,69 | 12,19 | 24,2 | 45000 |
| ICODEXTRINE | 7,54 | 34,32 | 6,5 - 8 | 12000 - 20000 |

On remarque d'après les résultats obtenus que plus le taux de branchement (le taux de liaisons α-1,6) augmente, plus l'hydrolyse amylasique est diminuée.

Cette dernière est également dépendante du poids moléculaire. Ainsi, plus le taux de branchement est élevé et le poids moléculaire petit, moins la molécule est attaquée par l'amylase

Pour une utilisation en dialyse intrapéritonéale, les produits A et Z sont particulièrement adaptés et présentent une résistance nettement supérieure à l'icodextrine, ce qui signifie que ces produits présentent un avantage certain en termes de durée du pouvoir osmotique et de pouvoir glycémiant, pour un poids moléculaire similaire.

### Exemple 5

On prépare des solutions aqueuses de polymères hautement branchés conformes à l'invention, que l'on met en contact d'une amylase d'origine pancréatique et d'une amyloglucosidase intestinale (poudre acétonique d'intestin). L'hydrolyse est suivie dans le temps par mesure du glucose apparaissant dans le milieu réactionnel. Ce test permet d'évaluer la résistance des polymères à l'hydrolyse par les enzymes impliquées dans la digestion des glucides alimentaires, ce qui est un critère essentiel dans le choix d'un ingrédient alimentaire rentrant dans la composition de formulations à l'usage des sportifs ou destinées à la nutrition entérale et parentérale.

Plusieurs polymères conformes à l'invention sont testés en comparaison avec l'icodextrine, le glycogène, et une maltodextrine standard. Les polymères choisis sont les suivants :

Produits A tel que préparé conformément à l'exemple 3, produit Y tel que préparé conformément à l'exemple 2, et produit Y' préparé selon l'exemple 2 à partir d'un amidon riche en amylopectine traité à l'enzyme de branchement et ultrafiltré.

L'icodextrine est fabriquée conformément au brevet EP 667.356 cité dans la description. Le glycogène est un glycogène de foie de boeuf fourni par la société SIGMA-ALDRICH.

Un témoin maltodextrine standard est réalisé pour valider le modèle in vitro de digestion enzymatique.

Les conditions opératoires pour la digestion enzymatique sont les suivantes :

Peser environ 0,6g de produit à tester précisément.

Ajouter 150mL de tampon maléate de sodium pH 7 à 0,1mol /L.

Agiter jusqu'à la dissolution du produit.

Prélever 1,5mL de la solution obtenue.

Placer les flacons au bain-marie pendant 15 minutes, pour que la température de la solution soit de 37°C.

Ajouter 0,15g de pancréatine de porc.

Incuber à 37°C au bain thermostaté sous agitation pendant 30 minutes.

Réaliser des prélèvements de 1,5mL aux temps : 0min et 30 minutes.

Arrêter la réaction enzymatique en plaçant les prélèvements dans un bain à sec à 100°C, pendant 10 minutes.

Ajouter 0,15g de muqueuse intestinale de rat.

Incuber pendant 5 h30 à 37°C au bain thermostaté sous agitation.

Réaliser des prélèvements de 1,5mL toutes les 60

minutes aux temps 60 ;120; 180; 240; 300 ;330 et 360 minutes.

Arrêter la réaction enzymatique en plaçant les prélèvements dans un bain à sec à 100°C, pendant 10 minutes.

Doser le glucose sur les prélèvements, pour calculer le pourcentage d'hydrolyse du produit étudié.

Pour le dosage du glucose, on utilise la même méthode que dans l'exemple 4.

Les résultats sont consignés dans les tableaux ci-après :
1. cinétique d'apparition du glucose (en % libéré sur sec)

| | **Temps (en min)** | **MALTOD. standard** | **GLYCOGENE** | **A** | **Y** | **Y'** | **ICODEXTRINE** |
|---|---|---|---|---|---|---|---|
| Amylase pancréatique | 0 | 0 | 0 | 0 | 0 | 0 | 0 |
| | 15 | 0,79 | 2,87 | 2,61 | 2,17 | 3,90 | 3,35 |
| | 30 | 1,06 | 3,30 | 2,70 | 2,71 | 4,74 | 3,63 |
| Amylase intestinale | 60 | 20,88 | 12,62 | 9,77 | 13,71 | 16,31 | 15,09 |
| | 90 | 38,59 | 22,81 | 16,66 | 23,34 | 28,29 | 26,96 |
| | 120 | 52,07 | 31,41 | 23,17 | 32,17 | 40,28 | 37,86 |
| | 150 | 62,90 | 39,45 | 28,66 | 39,22 | 48,64 | 47,22 |
| | 180 | 70,83 | 46,04 | 32,75 | 44,52 | 57,84 | 55,88 |
| | 210 | 78,76 | 51,50 | 37,03 | 49,00 | 64,53 | 63,01 |
| | 240 | 83,78 | 56,09 | 39,64 | 52,39 | 68,71 | 69,01 |
| | 270 | 88,81 | 59,96 | 42,62 | 54,56 | 72,33 | 72,09 |
| | 300 | 91,18 | 62,40 | 45,50 | 57,27 | 75,96 | 76,28 |
| | 330 | 93,03 | 64,26 | 47,27 | 58,63 | 79,44 | 78,37 |
| | 360 | 94,36 | 65,84 | 51,64 | 60,80 | 81,11 | 80,89 |

2. Synthèse des résultats

| **PRODUITS TESTES** | **% de glucose libéré à 360 min.** | **Taux de liaisons α-1,6 en %** | **Masse Molaire (daltons)** |
|---|---|---|---|
| MALTODEXTRINE STANDARD | 94,36 | 0 | 3000-5000 |
| GLYCOGENE | 65,84 | 10 | 10⁶ - 10⁷ |
| A | 51,64 | 19,4 | 33000 |
| Y | 60,80 | 16,3 | 138000 |
| Y' | 81,11 | 7,9 | 133000 |
| ICODEXTRINE | 80,89 | 6,5 - 8 | 12000 - 20000 |

Les maltodextrines selon l'invention sont particulièrement adaptées pour une utilisation en nutrition pour sportifs ou plus généralement pour réguler la glycémie. Les produits A et Y selon l'invention permettent d'obtenir un pourcentage de libération de glucose compris entre 50 et 70%, soit une résistance à l'hydrolyse nettement supérieure aux maltodextrines classiques et comparable au glycogène, ce qui signifie que ces produits présentent un avantage certain en termes de pouvoir glycémiant et peuvent ainsi constituer avantageusement un substitut du glycogène puisqu'ils présentent des caractéristiques de digestion similaires.

## Revendications

1. Polymères solubles de glucose hautement branchés ayant une teneur en sucres réducteurs inférieure à 1 %, **caractérisés par le fait qu'**ils présentent :
- un taux de liaisons glucosidiques α-16 compris entre 12 et 30 %,
- un Mw, déterminé par diffusion de la lumière, d'une valeur comprise entre 0,3.10⁵ et 2.10⁵ daltons,
- une osmolalité, déterminée selon un test A d'une valeur comprise entre 1 et 15 mOsm/kg, le test A consistant à déterminer l'osmolalité d'une solution renfermant 100 g sec desdits polymères de glucose hautement branchés placés dans 1 kg d'eau, à l'aide d'un osmomètre MARK 3 de FISKE®ASSOCIATES.

2. Polymères selon la revendication 1, **caractérisés par le fait qu'**ils présentent :
- un Mw déterminé par diffusion de la lumière d'une valeur comprise entre 0,5.10⁵ et 1,5.10⁵ daltons,
- une osmolalité, déterminée selon un test A, au moins égale à 1 et inférieure à 2 mOsm/kg.

3. Polymères selon la revendication 1, **caractérisés par le fait qu'**ils présentent :
- un Mw, déterminé par diffusion de la lumière d'une valeur comprise entre 0,5. 10⁵ et 0,8.10⁵ daltons.
- une osmolalité, déterminée selon un test A, au moins égale à 2 et inférieure à 5 mOsm/kg,

4. Polymères solubles de glucose hautement branchés selon l'une ou l'autre des revendications 2 et 3, **caractérisés par le fait qu'**ils présentent entre 15 et 30 % de liaisons glucosidiques α-1,6.

5. Polymères selon la revendication 1, **caractérisés par le fait qu'**ils présentent :
- un Mw, déterminé par diffusion de la lumière d'une valeur comprise entre 0,3.10⁵ et 0,7.10⁵ daltons,
- une osmolalité, déterminée selon un test A, au moins égale à 5 et inférieure à 15 mOsm/kg.

6. Procédé de préparation des polymères selon l'une quelconque des revendications 1 à 5, **caractérisé par le fait que** l'on :
- prépare une suspension aqueuse d'amidon ou une solution de dérivé d'amidon d'une matière sèche au moins égale 1 % en poids, de préférence de 10 à 50 % en poids,
- traite ladite suspension ou ladite solution avec au moins une enzyme de branchement à une température comprise entre 25 et 80°C pendant une durée de 1 à 24 heures,
- fait agir sur la suspension ou sur la solution ainsi obtenue au moins une enzyme choisie dans le groupe constituée de la β-amylase, l'amyloglucosidase et l'α-transglucosidase,
- effectue un fractionnement à l'aide d'au moins une technique choisie dans le groupe des séparations sur membrane ou chromatographies, de manière à récupérer les fractions de haut poids moléculaire et celles de bas poids moléculaire,
- recueille les polymères de glucose hautement branchés correspondants aux fractions de haut poids moléculaire ainsi obtenues.

7. Procédé selon la revendication 6, **caractérisé par le fait que** l'enzyme de branchement est choisie dans le groupe constitué par les enzymes de branchement du glycogène, les enzymes de branchement de l'amidon et les mélanges quelconques de ces enzymes.

8. Procédé selon l'une ou l'autre des revendications 6 et 7, **caractérisé par le fait que** la technique de fractionnement est choisie dans le groupe constitué par la technique de séparation sur membrane d'ultrafiltration et par la technique de séparation chromatographique sur support de type gel.

9. Utilisation des polymères de glucose hautement branchés selon l'une quelconque des revendications 1 à 5, pour préparer des compositions destinées à être utilisées dans les industries notamment du Papier-Carton, du Textile, de la Cosmétique, et en particulier dé la Pharmacie et de l'Alimentaire.

10. Utilisation selon la revendication 9, dans laquelle les compositions sont destinées à être utilisées dans les domaines de la nutrition entérale et parentérale, comme agent inhibiteur et régulateur de la glycémie, comme apport énergétique lors d'activités physiques et comme agent régulateur de la digestion.

11. Solution pour dialyse péritonéale **caractérisée en ce qu'**elle comprend en tant qu'agent osmotique au moins un polymère soluble hautement branché selon la revendication 1.

12. Solution pour dialyse selon la revendication 11, **caractérisée en ce que** ledit polymère soluble hautement branché présente :
- un Mw, déterminé par diffusion de la lumière d'une valeur comprise entre 0,3.10⁵ et 2.10⁵ daltons,
- une osmolalité, déterminée selon un test A, au moins égale à 5 et inférieure à 15 mOsm/kg.

13. Solution pour dialyse péritonéale selon l'une ou l'autre des revendications 11 et 12, **caractérisée en ce qu'**elle comprend en outre un polyol choisi dans le groupe constitué par le sorbitol, le mannitol, le maltitol, le xylitol, l'érythritol.

14. Solution selon la revendication 13, **caractérisée en ce qu'**elle comprend en outre des solutions tampons tels que les sels de lactate, d'acétate, de gluconate.

## Patentansprüche

1. Lösliche hochverzweigte Glukose-Polymere mit einem Gehalt an reduzierendem Zucker von weniger als 1%, **dadurch gekennzeichnet, dass** diese aufweisen:
- einen Gehalt an glykosidischen Bindungen α-1,6 zwischen 12 und 30%,
- ein Mw, bestimmt durch Lichtstreuung, zwischen 0,3.10⁵ und 2.10⁵ Dalton,
- eine Osmolalität, bestimmt gemäß einem Test A, zwischen 1 und 15 mOsm/kg, wobei der Test A darin besteht, die Osmolalität einer Lösung mit 100 g trocken der hochverzweigten Glukose-Polymere, welche in 1 kg Wasser angeordnet sind, mittels einem Osmometer MARK 3 von FISKE® ASSOCIATES zu bestimmen.

2. Polymere gemäß Anspruch 1, **dadurch gekennzeichnet, dass** diese aufweisen:
- ein Mw, bestimmt durch Lichtstreuung, zwischen 0,5. 10⁵ und 1,5. 10⁵ Dalton,
- eine Osmolalität, bestimmt gemäß dem Test A, von wenigstens gleich 1 und kleiner als 2 mOsm/kg.

3. Polymere gemäß Anspruch 1, **dadurch gekennzeichnet, dass** diese aufweisen:
- ein Mw, bestimmt durch Lichtstreuung, zwischen 0,5. 10⁵ und 0,8. 10⁵ Dalton,
- eine Osmolalität, bestimmt gemäß dem Test A, von wenigstens gleich 2 und kleiner als 5 mOsm/kg.

4. Lösliche hochverzweigte Glukose-Polymere gemäß einem der Ansprüche 2 und 3, **dadurch gekennzeichnet, dass** diese zwischen 15 und 30% an glykosidischen Bindungen α-1,6 aufweisen.

5. Polymere gemäß Anspruch 1, **dadurch gekennzeichnet, dass** diese aufweisen:
- ein Mw, bestimmt durch Lichtstreuung, zwischen 0,3. 10⁵ und 0,7. 10⁵ Dalton,
- eine Osmolalität, bestimmt gemäß dem Test A, von wenigstens gleich 5 und kleiner als 15 mOsm/kg.

6. Verfahren zur Herstellung von Polymeren gemäß einem der Ansprüche 1 bis 5, **dadurch gekennzeichnet, dass**:
- eine wässrige Suspension aus Stärke oder eine Lösung aus einem Stärkederivat einer Trockensubstanz von wenigstens gleich 1 Gew.-%, vorzugsweise 10 bis 50 Gew.-%, hergestellt wird,
- die Suspension oder die Lösung mit einem Verzweigungsenzym bei einer Temperatur zwischen 25 und 80°C während einer Dauer von 1 bis 24 Stunden behandelt wird,
- auf der Suspension oder auf der so erhaltenen Lösung wenigstens ein Enzym, das aus der Gruppe gewählt ist, die aus β-amylase, Amyloglukosidase und α-Transglukosidase gebildet ist, zum Agieren gebracht wird,
- eine Fraktionierung durchgeführt wird mittels wenigstens einer Technik, die aus der Gruppe von Trennungen auf Membran oder Chromatographien gewählt ist, um die Fraktionen mit hohem Molekulargewicht und jene mit niedrigem Molekulargewicht zu erhalten,
- die hochverzweigten Glukose-Polymere gewonnen werden, die den so erhaltenen Fraktionen mit hohem Molekulargewicht entsprechen.

7. Verfahren gemäß Anspruch 6, **dadurch gekennzeichnet, dass** das Verzweigungsenzym gewählt ist aus der Gruppe, die aus den Verzweigungsenzymen von Glykogen, den Verzweigungsenzymen von Stärke und beliebigen Mischungen dieser Enzyme besteht.

8. Verfahren gemäß einem der Ansprüche 6 und 7, **dadurch gekennzeichnet, dass** die Fraktionierungstechnik gewählt ist aus der Gruppe, die aus der Trennungstechnik auf Ultrafiltrationsmembran und chromatographischer Trennungstechnik auf einem Träger der Art Gel besteht.

9. Verwendung von hochverzweigten Glukose-Polymeren gemäß einem der Ansprüche 1 bis 5, , um Zusammensetzungen herzustellen, die dazu bestimmt sind, insbesondere in der Papier/Pappindustrie, Textilindustrie, Kosmetikindustrie und ganz besonders in der Pharmazie und Nahrungsmittelindustrie verwendet zu werden.

10. Verwendung gemäß Anspruch 9, bei welcher die Zusammensetzungen dazu bestimmt sind, in den Bereichen der enteralen und parenteralen Ernährung, als Inhibitor oder Regulator für die Glykämie, als Energiezufuhr bei körperlichen Aktivitäten und als Regulator für die Verdauung verwendet zu werden.

11. Lösung für Peritonealdialyse, **dadurch gekennzeichnet, dass** diese als Osmosemittel wenigstens ein lösliches hochverzweigtes Polymer gemäß Anspruch 1 aufweist.

12. Lösung für Dialyse gemäß Anspruch 11, **dadurch gekennzeichnet, dass** das lösliche hochverzweigte Polymer aufweist:
- ein Mw, bestimmt durch Lichtstreuung, zwischen 0,3. 10⁵ und 0,7.10⁵ Dalton,
- eine Osmolalität, bestimmt gemäß einem Test A, von wenigstens gleich 5 und kleiner als 15 mOsm/kg.

13. Lösung für Peritonealdialyse gemäß einem der Ansprüche 11 und 12, **dadurch gekennzeichnet, dass** diese des Weiteren ein Polyol aufweist, dass aus der Gruppe gewählt ist, die aus Sorbitol, Mannitol, Maititol, Xytitol, Erythritol gebildet ist.

14. Lösung gemäß Anspruch 13, **dadurch gekennzeichnet, dass** diese des Weiteren Pufferlösungen aufweist wie die Salze von Lactat, Acetat, Gluconat.

## Claims

1. Soluble highly branched glucose polymers having a reducing sugar content of less than 1%, having:
- a content of α-1,6 glucoside bonds of between 12 and 30%,
- a Mw value, determined by light scattering, of between 0.3x10⁵ and 2x10⁵ daltons,
- an osmolality value, determined according to a test A, of between 1 and 15 mOsm/kg, said test A consisting in determining the osmolality of a solution containing 100 g on a dry basis of highly branched glucose polymers that are in 1 kg of water using a fisk® associates MARK 3 osmometer.

2. Polymers according to Claim 1, **characterized in that** they have:
- a Mw value, determined by light scattering, of between 0.5x10⁵ and 1.5x10⁵ daltons,
- an osmolality, determined according to a test A, at least equal to 1 and less than 2 mOsm/kg.

3. Polymers according to Claim 1, **characterized in that** they have:
- a Mw value, determined by light scattering, of between 0.5x10⁵ and 0.8x10⁵ daltons,
- an osmolality, determined according to a test A, at least equal to 2 and less than 5 mOsm/kg.

4. Soluble highly branched glucose polymers according to any one of Claims 2 and 3, **characterized in that** they have between 15 and 30% of α-1,6 glucoside bonds.

5. Polymers according to Claim 1, having:
- a Mw value, determined by light scattering, of between 0.3x10⁵ and 0.7x10⁵ daltons,
- an osmolality, determined according to a test A, at least equal to 5 and less than 15 mOsm/kg.

6. Method for preparing the polymers according to any one of Claims 1-5, comprising the following steps:
- an aqueous starch suspension or a solution of starch derivative having a dry matter content at least equal to 1% by weight, preferably from 10 to 50% by weight is prepared,
- said suspension or said solution is treated with at least one branching enzyme at a temperature comprised between 25 and 80°C for a period of 1 to 24 hours,
- at least one enzyme selected chosen trom the group consisting of β-amylase, amyloglucosidase and α-transglucosidase is caused to act on the suspension or on the solution thus obtained,
- a fractionation using at least one technique selected from the group consisting of membrane separations or chromatographies is carried out, so as to recover the high molecular weight fractions and low molecular fractions,
- collecting the highly branched glucose polymers corresponding to the high molecular weight fractions thus obtained.

7. Method according to Claim 6, wherein the branching enzyme is selected from the group consisting of glycogen branching enzymes, starch branching enzymes and any mixtures of these enzymes.

8. Method according to any one et Claims 6-7, wherein the fractionation technique is selected from the group consisting of the technique of separation on an uftrafiltration membrane and the technique of chromatographic separation on a gel type support.

9. Use of highly branched glucose polymers according to any one of Claims 1 to 5, intended to be used in industries such as Paper-Carton, Textiles, Cosmetics, and especially Pharmaceutical and Food industries.

10. Use according to Claim 9, wherein the compositions are intended to be used for enteral and parenteral nutrition applications, as a glycemia inhibiting and regulating agent for energy source during physical activities and as a digestion regulating agent.

11. Solution for peritoneal dialysis, **characterized in that** it comprises, as osmotic agent, at least one soluble highly branched polymer according to Claim 1.

12. Dialysis solution according to Claim 11, wherein said soluble highly branched polymer has:
- a Mw value, determined by light scattering, of between 0.3x 10⁵ and 0.7x 10⁵ daltons,
- an osmolality, determined according to a test A, at least equal to 5 and less than 15 mOsm/kg.

13. Solution for peritoneal dialysis according to any one of Claims 11-12, additionally comprising a polyol selected from the group consisting of sorbitol, mannitol, maltitol, xylitol and erythritol.

14. Solution according to Claim 13, additionally comprising buffer solutions such as lactate, acetate and gluconate salts.
